# EUROPEAN PATENT SPECIFICATION

(11) **EP 3 053 625 B1**
(45) Date of publication and mention of the grant of the patent: **14.09.2022**
(21) Application number: 16154625.4
(22) Date of filing: 08.02.2016
(51) Int. Cl.: A61M 25/01, A61M 19/00

(54) **SYSTEM FOR INTERVENTIONAL NAVIGATION WITH A MEDICAL DEVICE AND MEDICAL DEVICE THEREFOR**
SYSTEM ZUR INTERVENTIONELLEN NAVIGATION MIT EINER MEDIZINISCHEN VORRICHTUNG
SYSTÈME DE NAVIGATION INTERVENTIONNELLE AVEC UN DISPOSITIF MÉDICAL ET DISPOSITIF MÉDICAL CORRESPONDANT

(30) Priority: 06.02.2015 EP 15154187
(43) Date of publication of application: 10.08.2016
(73) Proprietor: Magnebotix AG, 8952 Schlieren (CH)
(72) Inventor: BELL, Dominik, 8952 Schlieren (CH); OEZCAN, Mert, 8952 Schlieren (CH); FRUTIGER, Dominic, 8952 Schlieren (CH)
(74) Representative: Kley, Hansjörg

(56) References cited:
- EP-A1- 2 620 109
- WO-A2-2008/003059
- DE-A1-102010 043 481
- US-B1- 6 173 199

## Description

### TECHNICAL FIELD

The present invention relates to a system for performing a medical intervention in a subject's body, or in other words a system for interventional navigation with a medical device, wherein the medical device comprises several segments to be navigated through the body lumens or cavities, an external magnet and a control unit connected with the external magnet adapted to generate control signals for the external magnet to generate a magnetic field to orient a magnetically responsive element in the distal end portion of the medical device, especially to cause the distal end of the medical device to bend in a given direction for improving navigation.

### PRIOR ART

A variety of techniques are available to physicians for advancing elongated medical devices such as catheters, endoscopes and other surgical tools within a patient's body. When such an elongated medical device is advanced deeper within the patient's body, the advancing device becomes correspondingly more difficult to control, especially when the distal end has to corner sharply. However, especially during some medical procedures such as cardiac interventions, it is important that the advancing of the elongated medical device occurs in a very controlled and precise manner where the risk of a perforation or injury of the patient is minimized. It is also important that the physician's fatigue during the medical procedure is minimized.

A system according to the preamble of claim 1 is known from EP 2 620 109 A1 and is related to a magnetic clot disruptor comprising a magnetic wire, to be used to disrupt a clot within a body vessel. The wire includes a magnet that is positioned within the patient's body adjacent the clot. A magnetic driver is positioned outside the patient's body in proximity to the magnet. When the magnetic field of the magnetic driver is alternated, the wire moves within the body vessel to break up the clot. The control unit alternating the magnetic field switches the direction of the magnetic field between two directions, but since the distal end is positioned near the clot to be destroyed, it is pressed laterally into the clot and breaks it up. No further steering function is disclosed.

US 8,551,109 B2 is related to an apparatus and method for interventional navigation within a subject's body in which a medical device having at least one electrostrictive element is adapted to cause the distal end of the medical device to bend in a given direction for improving navigation. The medical device may further comprise at least one magnetically responsive element on the distal end, which may be oriented in the approximate direction of a magnetic field that is applied to the subject's body. At least one method for navigating a medical device though a subject's body is provided, by changing the direction of an applied magnetic field to align a magnetically responsive element on the distal end for orienting the distal end, and by applying a voltage to at least one electrostrictive element disposed in the distal portion of the device for causing the distal end to change orientation from that achieved by application of the magnetic field alone.

Therefore the effect of the external magnetic field is supported and enhanced by an additional force factor provided by electrostrictive elements in the distal end of the medical device. The improvement according to this prior art complicates the work of a physician since he has to control two electro-magnetic actuators, one external magnetic field and one voltage directly acting on the electrostrictive element.

DE 10 2010 043 481 A discloses a medicine system with aligning control unit that controls the relative position of a magnetic element such that the field axis is parallel to the rotational axis of the magnetic element. The medical system comprises a position detection system and a control unit. The position detection system detects the relative position between the rotation axis of the magnetic element and the field axis. The magnetic field is rotated in a direction that the power or the full torque is fully available for the rotation of the magnetic element.

WO 2008/003059 relates to a further electrostriction device and method for assisted magnetic navigation by bending the distal end of the medical device comprising a magnetically responsive element in a given direction for improving navigation. A method for navigating a medical device though a subject's body changes the direction of an applied magnetic field to align the magnetically responsive element on the distal end for orienting the distal end, and by applying a voltage to at least one electrostrictive element disposed in the distal portion of the device the distal end changes orientation from that achieved by application of the magnetic field alone.

US 6,173,199 B1 is related to a catheter having a distal end portion including a sensor responsive to the presence of a magnetic field or flux of a predetermined strength. The sensor is selected to respond when the distal end portion of the catheter is exposed to a magnetic field, such as provided by the external magnet, sufficient to capture and maneuver the distal end of the catheter.

### SUMMARY OF THE INVENTION

It is an object of the present invention to provide an improved system for orienting an elongated medical device, in particular a catheter, which allows for a precise movement based on a single electro-magnetic effector and avoids negative impact from the misalignment between the magnetic field and the magnetic distal end of the medical device, which can, as will be explained in connection with the drawings, lead to a torque based flip of the distal end of the and thus to damaging nearby lumen walls.

This object is achieved by the system according to claim 1 by a system for performing a medical intervention in a subject's body, comprising a medical device comprising several segments to be navigated through the body lumens or cavities, one or more external magnets and a control unit connected with the external magnet(s) adapted and configured to generate control signals for the external magnet(s) to generate a magnetic field having a direction to orient a magnetically responsive element in the distal end portion of the medical device, especially to cause the distal end portion of the medical device to bend in a given direction for improving navigation.

In other words the magnetic field generated through control signals from the control unit has a specific direction which is - in the control mode - not parallel to the distal end of the medical device. If it is, at a point in time, parallel, then no more bending takes place and the distal end is directed in the direction as given by the control unit. Usually, the navigation comprises an advancing (or retreating) of the medical device. The medical deivce is usually a catheter with a long proximal part extending through e.g. the blood vessel system of a patient. The orientation of proximal part is at that time no longer interesting, since the navigation issues are related to the distal part. There an initial, sometimes incremental, advance always takes place in the direction of the magnetic field. Then the advance necessitates a direction change. The person using the system uses the control unit for the external magnetic field to change the direction of this magnetic field, e.g. diverts the direction of the magnetic field from the parallel alignment with the distal end.

This control change can be performed via a switch, if the new orientation in the 3D space is known and predetermined, or the control change can be performed by incremental changes of the angle of the magnetic field, starting from an alignment with the distal end. These changes starting from the direction defined by the direction of the distal end can be limited to a specific plane or open the entire cone with the cone tip virtually located at the bending point or a portion of the distal end, i.e. in its prolongation.

According to the invention, the control unit is adapted and configured to allow to limit the generation of control signals for the external magnet in a way that the direction of the new magnetic field generated after the accomplishment of the orientation change of the magnetic field is limited within a field limiting frame around the longitudinal axis of the distal end portion of the medical device.

In other words, the control unit, being adjusted to a first magnetic field direction, preferably in the direction of the distal end, now receiving a control input defining a second magnetic field direction generates control signals for the magnetic field orientation, e.g. turning the external magnets or controlling the overlapping magnetic field of several electric magnets, only up to a second magnetic field direction which is inside the predetermined field limiting frame.

The control unit is configured to store a first direction, especially the current direction of the magnetic field or the current direction of the distal end, as starting direction for the following limitation step. The control unit is configured, e.g. through lapse of a time interval or by initiation by a user, to store this first direction temporarily until the next separated bending and advancing step. This storage action can be initiated in common or separately by fixing a field limiting frame for the external magnet. The fixation of the field limiting frame for the external magnet comprises to allow the generation of control signals for the external magnet to determine the direction of the magnetic field in a way that the direction of the magnetic field is limited within such a field limiting frame around the stored first direction, i.e. the longitudinal axis of the distal end portion of the medical device at the time of initiating the present bending and advancing step. That is, that the central axis of the cone is predetermined to be in line with the longitudinal axis direction of the magnetic tip or the distal end, respectively of the medical device, at that initiating step.

The control unit is thus configured to store the current direction of the distal end portion or the current direction of the magnetic field as stored direction and to subsequently limit the generation of control signals for the external magnet(s) in a way that the direction of the magnetic field is limited within a field limiting frame around the previously stored direction.

It is preferred that the user of the system can give said calibrating and limiting order by e.g. pushing a button or, if after a specific time of having oriented the distal end in a first direction, the calibration takes automatically place in said first direction and the limitating frame is defined when the user diverts from the original first direction towards a second direction having an angle in comparison to the first direction being beyond a predefined threshold angle, e.g. 2 or 5 degrees.

It is of course possible that there is not only one external magnet; the system can comprise two or more external magnets.

In an embodiment of the system, the field limiting frame is a cone. It can have an inverted regular form having a regular constant or adjustable opening angle around the central axis of the cone. It can be taken from the group of base surfaces being a circle, an ellipse, the shape of a bone or another simple closed curve.

A further embodiment of the system has a central axis of the cone which is predetermined to be in line with the longitudinal axis direction of the magnetic tip or the distal end, respectively of the medical device.

The control unit can comprise a switch or button to enable or disable the field limiting frame. Then it switches the limiting opening angle on or off. Furthermore it can be provided to adjust the limiting opening angle from e.g. 90 degree to 140 degree divergence to the first direction.

A medical device, especially for use within a system according to the invention, comprises beside the magnetic tip at least one further magnetic segment with non-magnetic flexible shaft portions between magnet segments(s) and/or the magnetic tip, respectively, wherein optionally the field limiting frame is provided with its main axis along the orientation of the magnetic tip.

A method for moving the distal end of an elongated medical device, in particular a catheter, by means of a magnetic field, is provided. A method of performing a medical intervention in a subject's body, comprises navigation of at least one medical device comprising several segments through the body lumens or cavities, the navigation method comprising the step of applying a magnetic field to orient a magnetically responsive element in the distal end portion of the medical device and advancing the device, and further comprising the step of storing the current direction of the distal end portion or the current direction of the magnetic field as stored direction and to limit in a subsequent navigation step the generation of control signals for the external magnet(s) in a way that the direction of the magnetic field is limited within a field limiting frame around the previously stored direction. This relates to limiting the direction of the magnetic field within a field limiting frame around the longitudinal axis of the distal end portion of the medical device for any subsequent bending and/or navigating step. Navigating steps are steps where the medical device is pushed or retracted, i.e. performs a longitudinal movement.

Such a method can, according to an embodiment, combine the limiting function with an ablation step wherein the direction of the magnetic field is rotated along the borders of the field limiting frame. This can be done by a rotation along the bordering surface of an inverted cone, wherein the apex is on the main direction of the distal end portion.

Further embodiments of the invention are laid down in the dependent claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a schematical side view of a distal end of a medical device influenced by an external magnetic field;
- Fig. 2: shows a schematical side view of the distal end of the medical device of Fig. 1 influenced by a magnetic field having a higher misalignment with the distal tip than the field of Fig. 1;
- Fig. 3: shows a schematical side view of the distal end of the medical device of Fig. 1 influenced by a magnetic field with even a higher misalignment than the field of Fig. 2 and leading to a flip of the distal tip of the medical device;
- Fig. 4: shows a schematical side view of the distal end of the medical device of Fig. 1 influenced by a magnetic field with a field reference frame avoiding a flip of the distal end of the medical device;
- Fig. 5: shows a side view of the distal end of the medical device of Fig. 1 touching a lumen wall;
- Fig. 6: shows the situation of Fig. 5 after a slipping incident;
- Fig. 7: shows the preparatory situation and position of a medical device before a circular ablation of vessel walls;
- Fig. 8: shows the advantage of applying the limiting field alignment in the situation of Fig. 7; and
- Fig. 9: shows a specific multi-segment medical device according to a further embodiment of the invention.

### DESCRIPTION OF PREFERRED EMBODIMENTS

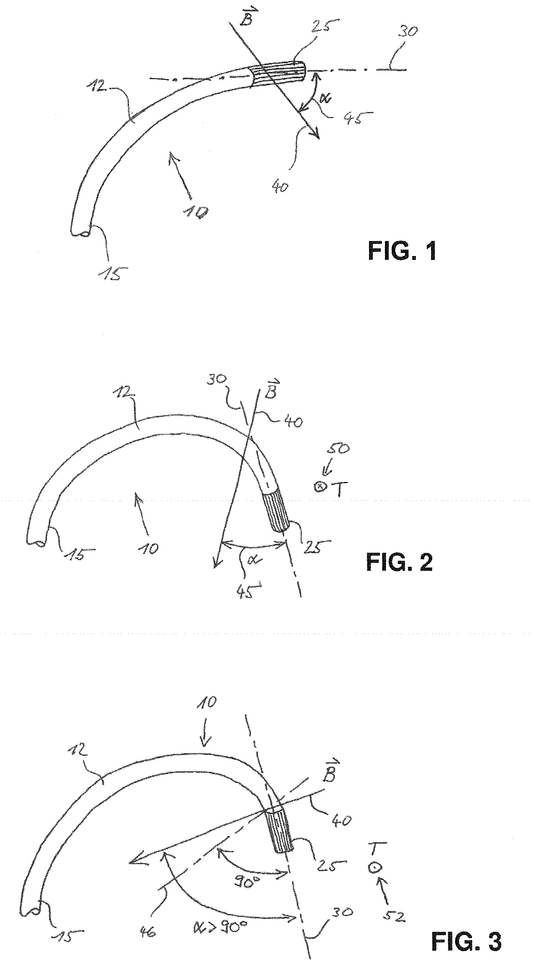

Fig. 1 to Fig. 3 show schematical side views of a distal end 20 of a medical device 10 influenced by a magnetic field B having the direction of arrow 40. The distal end 20 is essentially straight and has one well defined direction 30 along its longitudinal axis. The medical device 10 further comprises a flexible shaft 12 allowing the bending of the distal end 20 compared to the more distant portions 15 of the medical device W, 10.

The distal end 20 comprises a magnetic tip 25. The magnetic tip 25 can be a solid or a sleeve around a core of the central medical device 10; it comprises preferably ferromagnetic material but can be composed of any material which can be influenced by the external magnetic field B having the direction 40. Magnetic tip 25 is not necessarily at the free end of the medical device 10, but can also be provided in a short distance. The length of the magnetic tip 25 can have e.g. a length of between 5 and 20 millimeter while having a diameter of e.g. 3 to 8 millimeter.

Fig. 2 shows the medical device 10 of Fig. 1 influenced by a magnetic field with an orientation according to arrow 40 having a higher misalignment angle 45 than the misalignment angle between the field 40 and the longitudinal direction 30 of the magnetic tip 25 of Fig. 1. Reference numeral 50 indicates the torque T influencing the distal tip 25 to follow the direction 40 of magnetic field B leading to a higher bend and curvature of the distal end 20 of the medical device 10. In other words, the misalignment angle between field direction 40 and magnetic tip 25 increases with increasing device deformation, e.g. of a catheter, since even if the medical device 10 is not stiff, it has an increasing resistance against bending with higher bending angles.

In different situations, a physician is nevertheless obliged to bend the distal portion 20 of the medical device 10 even more to e.g. direct the tip of the medical device into a blood vessel having an angle of e.g. more than 90 or 120 degrees in view of the larger vessel, where the not so distant parts 15 of the medical device 10 are provided. Fig. 3 shows such a situation where the magnetic tip 25 should even bend more. The misalignment angle 45 between the direction 40 of the magnetic field Band the direction 20 of the distal tip 20 is higher than 90 degree, whereas the right angle is shown with the axis as dotted line 46 being perpendicular to the general direction 30 of the distal tip 25 of the medical device 10. In such a case the direction of torque T according to the arrow 52 flips over and the distal tip 20, instead of bending further and following the direction of the magnetic field B in its direction 40, it flips over to the opposite direction. This might be dangerous especially if there is a vessel wall on the other side, where the flip occurs. The situation is problematic for the physician, since the distal tip 20 may be scraping along a lumen wall and when freed, flip over. Another problem resides in the fact that the physician has to direct the distal tip 20 in a 3D environment and the misalignment angle 45 in the 2D representation as in Fig. 1 to 3 may misguide him in the evaluation of the effective angle of misalignment 45. Additionally the direction of magnetic field 30 is not visible as such.

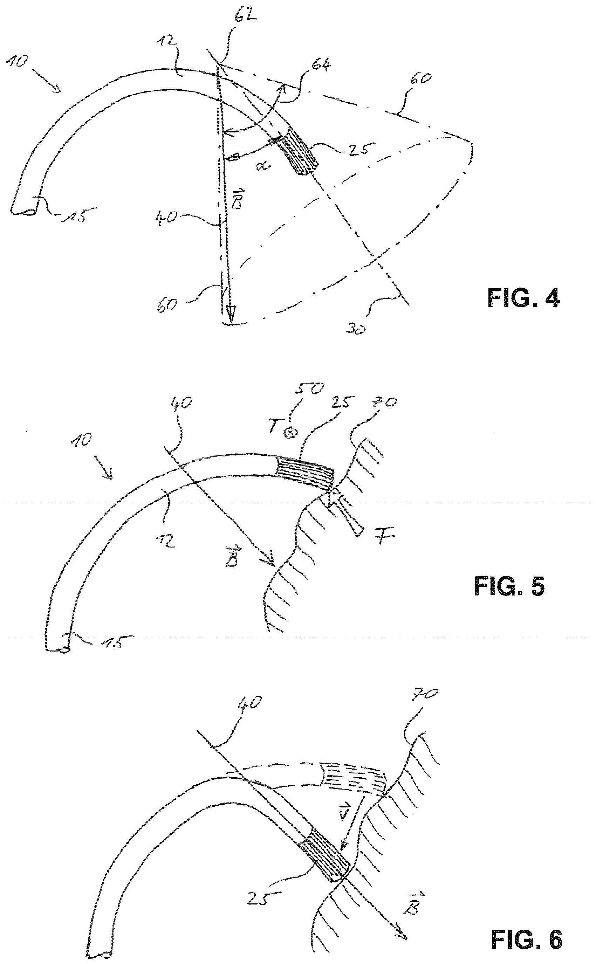

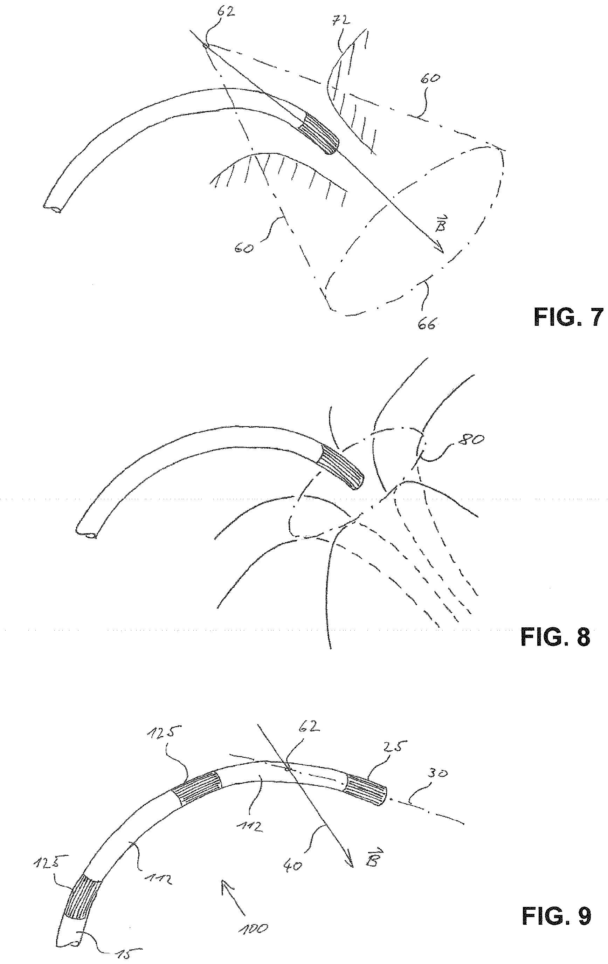

Fig. 4 shows a schematical side view of the distal end of the medical device of Fig. 1 influenced by a magnetic field B having the orientation 40 with a field limiting frame 60 avoiding a flip of the medical device 10. The field limiting frame 60 is shown as two lines opening a cone. The cone is a three-dimensional geometric shape that widens from a point 62 called the apex or vertex to a virtual base (frequently, though not necessarily, circular, as shown in Fig 7). It is preferred that the cone is an inverted regular form having a regular constant opening angle 64 around the central axis of the cone. The axis of the cone is the straight line, passing through the apex, about which the virtual base (not shown) and the whole cone has a rotational symmetry and which is in line with the longitudinal axis direction 30 of the magnetic tip 25 or the distal end 20, respectively. This is the cone according to the embodiment shown in Fig. 4. Of course, it is not necessary that the cone is a regular constant cone, it can have a changing opening angle, e.g. the opening angle can provide not a circle as base line but an ellipse or another form; it is also not necessary that the surface of the cone is always convex; it can be any simple closed curve. Furthermore it is possible that the cone form can be chosen within the control unit beforehand, i.e. the cone angle is adjustable. This can also comprise a switch between different cone forms, e.g. between a circular base to an elliptic base to a base having the "shape of a bone" or any other simple closed curve.

In view of the direction 40 of the magnetic field B, the field limiting frame 60 can directly be applied to the axis 30 of the magnetic tip 25. The magnetic field is controlled through a control unit predetermining the strength and the direction 40 of the magnetic field B. The system according to an embodiment of the invention comprises a field limiting function. Said field limiting function can comprise a control button providing a value of a field limiting frame 60 comprising an opening angle fixed in the 3D space around the direction 30 of the distal tip 25 at the moment of actuation of this control button or after the actuation of the control button.

In other words, the physician evaluates the position of the distal tip 20 and the existing pre-bending condition, e.g. before he evaluates next advancing and bending steps of the medical device 10, if he has to turn the tip 20 of the medical device 10 into a new direction without being guided by the walls of a lumen. According to prior art devices, the physician makes the decision that the turning movement will be feasible without risking the situation of Fig. 3. According to the invention, the system allows to fix the limiting directions for the magnetic field direction 30 through fixing its opening angle through the field limiting frame 60. Then he can advance the medical device 10 and bending the distal tip 20 through manipulating the direction 40 and or strength of the magnetic field B, since the misalignment angle 45 is limited by the predefined limiting cone angle 64. The field limiting frame 60 angle 64 can be e.g. 90 to 140 degrees.

In other words, the distal tip 20 is oriented along a first direction 30 through former advancing steps. This can comprise an ongoing bending as shown in Fig. 4 with a bent flexible shaft 12. Nevertheless, Fig. 4 shows a specific point in time before the next control step. Either the control unit senses through measuring a time interval via a threshold of non-action that the current first direction 30 of the distal end is to be taken as originating direction or the user of the system pushes a button to define said first direction 30. Then the physician makes the decision to turn the distal end for the next treatment or advance step. The field frame limiting decision is either made by the physician through actuation of a button or automatically from the predefined first direction. The control unit is in either case built and configured to allow generation of control signals for the orientation of the external magnet in a way that the distal end 20 can - after execution of the new orientation of the external magnet - only be oriented up to a second new direction which is limited by the directions of the field limiting frame 60 or cone around the longitudinal axis of the distal end portion of the medical device prior to the new orientation, which is stored at said calibration step in the control unit for this navigation or bending step.

In other words, the new direction of the distal tip 25 after the bending is at most the limiting directions provided by the field limiting frame 60 as e.g. a cone, or any direction in between.

Fig. 5 shows a side view of the distal end 20 of the medical device 10 of Fig. 1 touching a lumen wall 70. Fig. 6 shows the situation of Fig. 5 after a slipping incident, where the final position of the magnetic tip 25 is limited by the (maximum) opening angle of the field limiting frame 60. Here the physician recognizing that the tip 25 touches the lumen wall 70 and blocks the motion in the situation of Fig. 5 before trying to move the distal end 20. When the slipping incident occurs, the orientation 30 of the distal end 20 cannot go beyond the orientation of the magnetic field B when the direction 40 of the magnetic field B is in line with the angle opening line of the cone as defined before the bending step at the limit engaging step at Fig. 4 through storing the former direction 30 as first direction and defining direction for the field limiting frame or cone 60 for any new second direction.

Fig. 7 shows the preparatory situation and position of a medical device 10 before a circular ablation 80 of vessel walls 72 and Fig. 8 shows the advantage of applying the field limiting frame 60 in the situation of Fig. 7. The medical device 10 is pushed and bent into the opening of e.g. a pulmonary vein, so that the direction 40 of the magnetic field B is in alignment with the direction 30 of the magnetic tip 25 of the medical device 10. Then the control unit is activated to fix the opening angle 64 of the field limiting frame 60; here the limited opening angle 64 can be chosen smaller so that the use of the maximum misalignment angle exerts a predetermined force. Then the circular ablation is effected and forms the circular ablation edge 80.

Fig. 9 shows a specific multi-segment medical device 100 according to a further embodiment of the invention. Beside the magnetic tip 25, at least one, here two further magnetic segments 125 are provided with inserted non-magnetic flexible shaft portions 112. The magnetic segments 125 can be solid segments with non-magnetic flexible shaft portions 112 attached or they can comprise a sleeve attached to a continuous non-magnetic flexible shaft material.

The segments 25, 125 can have a length of 8 to 15 millimeter, whereas the shaft portions 112 can have the same length or be up to 2 times longer.

The externally applied magnetic field can be at about 0.1 to 0.15 Tesla, to orient the magnetically responsive tip 25 and magnetic segments 125 of the medical device 10 or 100.

**LIST OF REFERENCE SIGNS**

| | | | |
|---|---|---|---|
| 10 | medical device | 30 | orientation of distal end |
| 12 | flexible shaft | 40 | orientation of magnetic field |
| 15 | distant portion | 45 | angle of misalignment |
| 20 | distal end | 46 | 90 degree angle |
| 25 | magnetic tip | 50 | direction of torque |
| 52 | direction of torque | 80 | ablation edge , circular ablation |
| 60 | field limiting frame | 100 | multi-segment medical device |
| 62 | apex | 112 | flexible shaft portion |
| 64 | opening angle | 125 | magnetic segment |
| 66 | virtual base of the cone | | |
| 70 | lumen wall | | |
| 72 | vessel walls | | |

## Claims

1. A system for performing a medical intervention in a subject's body, comprising a medical device (10) comprising several segments (12, 15, 25) to be navigated through the body lumens or cavities, one or more external magnets and a control unit connected with the external magnet(s) configured to generate control signals for the external magnet(s) to generate a magnetic field (B) having a direction (40) to orient a magnetically responsive element (25) in the distal end portion (20) of the medical device (10), causing the distal end portion (20) of the medical device (10) to bend in a given direction (30), **characterized in that**,
the control unit is configured to store the current direction of the distal end portion (20) or the current direction of the magnetic field (B) as stored direction and to subsequently limit the generation of control signals for the external magnet(s) in a way that the direction (40) of the magnetic field (B) is limited within a field limiting frame (60) around the previously stored direction of the longitudinal axis (30) of the distal end portion (20) of the medical device (10) for improved navigation.

2. The system according to claim 1, wherein the field limiting frame (60) is a cone, especially an inverted regular form having a regular constant or adjustable opening angle (64) around the central axis of the cone, especially taken from the group of base surfaces being a circle, an ellipse, the shape of a bone or another simple closed curve.

3. The system according to claim 2, wherein the central axis of the cone is predetermined to be in line with the longitudinal axis direction (30) of the magnetic tip (25) or the distal end (20), respectively of the medical device (10).

4. The system according to any of claims 1 to 3, wherein the control unit comprises a switch to enable or disable the field limiting frame (60).

5. The system according to claim 4, wherein enabling or disabling the field limiting frame (60) comprises to switch on or off the limiting opening angle (64).

6. The system according to any of claims 1 to 5, wherein the control unit comprises a switch to adjust the limiting opening angle (64).

7. The system according to one of claims 1 to 6, wherein the medical device (10, 100) comprises beside the magnetic distal end portion (20) of the medical device (10) at least one further magnetic segment (125) with non-magnetic flexible shaft portions (112) between magnet segments(s) and/or the magnetic distal end portion (20), respectively, wherein the field limiting frame (60) is provided with its main axis along the orientation (30) of the magnetic distal end portion (20).

## Patentansprüche

1. System zur Durchführung eines medizinischen Eingriffs im Körper eines Patienten, umfassend eine medizinische Vorrichtung (10), die mehrere Segmente (12, 15, 25) umfasst, die durch die Körperlumen oder Körperhohlräume zu navigieren sind, einen oder mehrere externe Magnete und eine Steuereinheit, die mit dem/den externen Magneten verbunden ist und so konfiguriert ist, dass sie Steuersignale für den/die externen Magneten erzeugt, um ein Magnetfeld (B) mit einer Richtung (40) zu erzeugen, um ein magnetisch reagierendes Element (25) im distalen Endabschnitt (20) der medizinischen Vorrichtung (10) auszurichten, wodurch der distale Endabschnitt (20) der medizinischen Vorrichtung (10) veranlasst wird, sich in eine bestimmte Richtung (30) zu biegen,
**dadurch gekennzeichnet, dass**,
die Steuereinheit so konfiguriert ist, dass sie die aktuelle Richtung des distalen Endabschnitts (20) oder die aktuelle Richtung des Magnetfelds (B) als gespeicherte Richtung speichert und anschließend die Erzeugung von Steuersignalen für den/die externen Magneten so begrenzt, dass die Richtung (40) des Magnetfelds (B) innerhalb eines Feldbegrenzungsrahmens (60) um die zuvor gespeicherte Richtung der Längsachse (30) des distalen Endabschnitts (20) der medizinischen Vorrichtung (10) für eine verbesserte Navigation begrenzt wird.

2. System nach Anspruch 1, wobei der Feldbegrenzungsrahmen (60) ein Kegel ist, insbesondere eine umgekehrte regelmäßige Form mit einem regelmäßigen konstanten oder einstellbaren Öffnungswinkel (64) um die Mittelachse des Kegels, insbesondere aus der Gruppe der Grundflächen, die ein Kreis, eine Ellipse, die Form eines Knochens oder eine andere einfache geschlossene Kurve sind.

3. System nach Anspruch 2, wobei die Mittelachse des Konus so vorgegeben ist, dass sie mit der Längsachsenrichtung (30) der Magnetspitze (25) bzw. des distalen Endes (20) des Medizinprodukts (10) fluchtet.

4. System nach einem der Ansprüche 1 bis 3, wobei die Steuereinheit einen Schalter zum Aktivieren oder Deaktivieren des Feldbegrenzungsrahmens (60) umfasst.

5. System nach Anspruch 4, wobei das Aktivieren oder Deaktivieren des Feldbegrenzungsrahmens (60) das Ein- oder Ausschalten des Begrenzungsöffnungswinkels (64) umfasst.

6. System nach einem der Ansprüche 1 bis 5, wobei die Steuereinheit einen Schalter zum Einstellen des Begrenzungsöffnungswinkels (64) umfasst.

7. System nach einem der Ansprüche 1 bis 6, wobei das medizinische Gerät (10, 100) neben dem magnetischen distalen Endabschnitt (20) des medizinischen Gerätes (10) mindestens ein weiteres Magnetsegment (125) mit nichtmagnetischen flexiblen Schaftabschnitten (112) zwischen Magnetsegmenten bzw. dem magnetischen distalen Endabschnitt (20) aufweist, wobei der Feldbegrenzungsrahmen (60) mit seiner Hauptachse entlang der Ausrichtung (30) des magnetischen distalen Endabschnittes (20) vorgesehen ist.

## Revendications

1. Système pour réaliser une intervention médicale dans le corps d'un patient, comprenant un dispositif médical (10) comprenant plusieurs segments (12, 15, 25) à faire naviguer dans le lumen ou dans les cavités du corps, un ou plusieurs aimants externes et une unité de commande connectée au(x) aimant(s) externe(s) configurée pour générer des signaux de commande pour le(s) aimant(s) externe(s) afin de générer un champ magnétique (B) ayant une direction (40) pour orienter un élément magnétiquement sensible (25) dans la partie d'extrémité distale (20) du dispositif médical (10), provoquant la flexion de la partie d'extrémité distale (20) du dispositif médical (10) dans une direction donnée (30),
**caractérisée en ce que**
l'unité de commande est configurée pour stocker la direction actuelle de la partie d'extrémité distale (20) ou la direction actuelle du champ magnétique (B) en tant que direction stockée et pour limiter ensuite la génération de signaux de commande pour le ou les aimants externes d'une manière telle que la direction (40) du champ magnétique (B) est limitée à l'intérieur d'un cadre de limitation de champ (60) autour de la direction précédemment stockée de l'axe longitudinal (30) de la partie d'extrémité distale (20) du dispositif médical (10) pour une meilleure navigation.

2. Système selon la revendication 1, dans lequel le cadre de limitation de champ (60) est un cône, notamment une forme régulière inversée ayant un angle d'ouverture (64) régulier constant ou réglable autour de l'axe central du cône, notamment pris dans le groupe des surfaces de base étant un cercle, une ellipse, la forme d'un os ou une autre courbe fermée simple.

3. Système selon la revendication 2, dans lequel l'axe central du cône est prédéterminé pour être en ligne avec la direction de l'axe longitudinal (30) de la pointe magnétique (25) ou de l'extrémité distale (20), respectivement du dispositif médical (10).

4. Système selon l'une quelconque des revendications 1 à 3, dans lequel l'unité de commande comprend un interrupteur pour activer ou désactiver le cadre de limitation de champ (60).

5. Système selon la revendication 4, dans lequel l'activation ou la désactivation du cadre de limitation de champ (60) consiste à activer ou désactiver l'angle d'ouverture limite (64).

6. Système selon l'une quelconque des revendications 1 à 5, dans lequel l'unité de commande comprend un interrupteur pour régler l'angle d'ouverture limite (64).

7. Système selon l'une des revendications 1 à 6, dans lequel le dispositif médical (10, 100) comprend, à côté de la partie d'extrémité distale magnétique (20) du dispositif médical (10), au moins un autre segment magnétique (125) avec des parties d'arbre flexibles non magnétiques (112) entre le ou les segments magnétiques et/ou la partie d'extrémité distale magnétique (20), respectivement, dans lequel le cadre de limitation de champ (60) est prévu avec son axe principal le long de l'orientation (30) de la partie d'extrémité distale magnétique (20).
